# EUROPEAN PATENT APPLICATION

(11) **EP 1 674 463 A1**
(43) Date of publication of application: **28.06.2006**
(21) Application number: 05026579.2
(22) Date of filing: 06.12.2005
(51) Int. Cl.: C07D 401/12, A61K 31/4439, A61P 1/04

(54) **Rabeprazole sodium salt in crystalline hydrate form**

(30) Priority: 21.12.2004 IT MI20042437
(71) Applicant: Dipharma S.p.A., 33036 Mereto di Tomba (Udine) (IT)
(72) Inventor: Malpezzi, Luciana, 20131 Milano (IT); Giovenzana, Tommaso, 20100 Milano (IT); Allegrini, Pietro, 20097 San Donato Milanese (MI) (IT); Ventimiglia, Gianpiero, 20092 Cinisello Balsamo (MI) (IT)
(74) Representative: Minoja, Fabrizio

(57) **Abstract**

Rabeprazole sodium in crystalline hydrate forms, a pharmaceutical composition containing them, their use in therapy, a process for their preparation, and the use thereof for the purification of rabeprazole sodium.

## Description

### FIELD OF THE INVENTION

The present invention relates to rabeprazole sodium in crystalline hydrate forms, a pharmaceutical composition thereof, their use in therapy, a process for their preparation and their use in a process for the purification of rabeprazole sodium.

### TECHNOLOGICAL BACKGROUND

Rabeprazole sodium, or 2-[[[4-(3-methoxyprvpoxy)-3-methyl-2-pyridinyl]-methyl]sulfinyl]-*1H*-benzilimidazole sodium salt, of formula 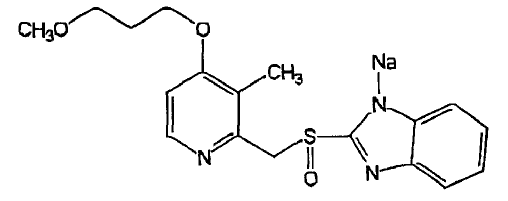 is an inhibitor of gastric secretion used for the treatment of peptic ulcer.

EP 268 956 discloses the preparation of rabeprazole sodium by crystallization from ethyl ether, in the form of white crystals, having m.p. 140-141°C (dec.). Four crystalline forms of rabeprazole sodium have been described to date, namely that referred to as Form II in JP 2001-39975, which is obtained starting from rabeprazole sodium in the solid, non-crystalloid form; those referred to as Forms X and Y in WO 03/082858; and that referred to as Form Z in US 2004/0180935. Different forms of biologically active compounds, particularly polymorphic forms, are known to be potentially useful in therapy, thanks to their different bioavailabilities, release times and solubilities. This can be greatly advantageous for patients, in that both a reduction in the drug dosage and a longer time between administrations can be attained. Furthermore, the different physical characteristics often connected with the various physical forms of the drug, such as hygroscopicity, flowability and/or compaction of the powders, can advantageously be used in the pharmaceutical technique for the preparation of pharmaceutical formulations.

There is therefore the need for novel polymorphic forms of biologically active compounds, which can provide advantageous properties in therapy and/or in pharmaceutical technique.

### SUMMARY OF THE INVENTION

It has now been found that rabeprazole sodium can exist, in addition to the above mentioned crystalline forms, also in crystalline hydrate forms, more particularly in the crystalline hydrate forms in the following referred to as Form α and Form β, stable at room temperature.

Therefore, the invention relates to rabeprazole sodium in crystalline hydrate forms, in particular as Form α and Form β, a method for their preparation, a pharmaceutical composition containing said forms and their use in therapy.

A further object of the invention is a process for the purification of rabeprazole sodium by using said crystalline hydrate Form α or Form β, to obtain rabeprazole sodium of suitable quality to fulfill the regulatory requirements for products for the therapeutical use.

### BRIEF DISCLOSURE OF THE FIGURES AND ANALYTIC METHODS

The novel crystalline hydrate Form α and Form β of rabeprazole sodium were characterized by X-ray powder diffraction (XRPD), ¹H-NMR nuclear magnetic resonance spectrometer and differential scanning calorimetry (DSC), The water content in the compounds was determined by titration according to the Karl Fisher technique. X-ray diffraction spectra (XRPD) were recorded with a θ/θ automatic diffractometer for powders and liquids manufactured by Ital-Structures, under the following operative conditions: radiation CuKα (λ = 1.5418 Å), scanning with angular step of 0.03° for a time of 2 sec. ¹H-NMR spectrum was recorded with a Varian Mercury 300 spectrometer, using DMSO-d6 as solvent. DSC thermogram was recorded with a Mettler-Toledo DSC 822e differential scanning calorimeter, under the following operative conditions: aluminium capsules, interval 30-400°C with 10°C/min speed, nitrogen as purging gas (80 ml/min).
Figure 1: XRPD spectrum of rabeprazole sodium Form α.
Figure 2: DSC thermogram of rabeprazole sodium Form α.
Figure 3: XRPD spectrum of rabeprazole sodium Form β.
Figure 4: DSC thermogram of rabeprazole sodium Form β.

### DETAILED DISCLOSURE OF THE INVENTION

A first object of the present invention is rabeprazole sodium in the crystalline hydrate form.

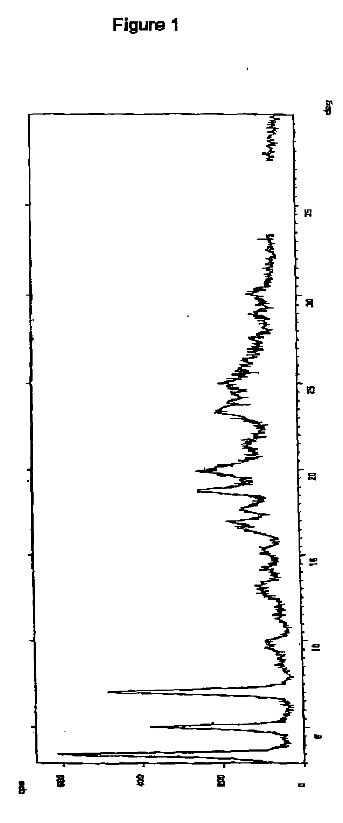

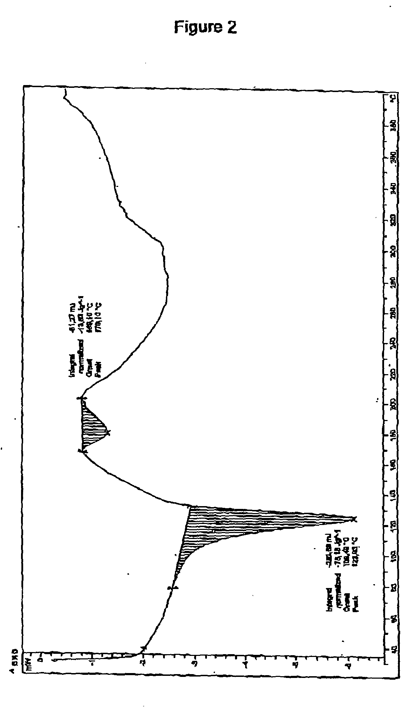

According to a preferred aspect of the invention, an hydrate form, in the following referred to as Form α, has water content ranging between 2.2 and 3.0% in weight, preferably between approximately 2.5 and 2.8% in weight, so that it can be defined as an approximately hemihydrate form. Said Form α, has a DSC thermogram substantially as reported in Figure 2, and an XRPD spectrum substantially as reported in Figure 1, wherein the more intense diffraction peaks are observed at 3.8; 5.1; 7.1; 16.9; 17.6; 18.8 and 19.9 ± 0.2° in 2θ.

Rabeprazole sodium in the crystalline Form α, can be prepared by a process comprising:
- dissolving a rabeprazole sodium dispersion in an organic polar aprotic solvent;
- keeping the solution at room temperature for a time equal to or higher than 24 hours; and
- recovering the resulting solid.

The process can be carried out starting from a dispersion of rabeprazole sodium in a polar aprotic solvent. The starting crude rabeprazole sodium can be obtained as disclosed e.g. in EP 268 956. Examples of aprotic polar solvents are lower carboxylic acid alkyl esters or mixtures thereof, typically of formula RCOOR', wherein R is hydrogen or C₁-C₄ alkyl and R' is C₁-C₄ alkyl. An alkyl group can be straight or branched. Preferred examples of solvents are ethyl acetate, butyl acetate, isopropyl acetate, ethyl propionate, isobutyl propionate and ethyl butyrate or mixtures of two or three thereof. More preferred are ethyl acetate, isopropyl acetate and butyl acetate, or mixtures thereof, in particular butyl acetate. The concentration of rabeprazole sodium in the starting solution can range approx. from 2 to 12% w/w, preferably approx. from 5 to 8.5% w/w. The temperature of the dispersion is then brought to a value higher than 20°C, preferably about 35-45°C, to completely dissolve rabeprazvle sodium. The resulting solution is left to stand at room temperature for 24 hours or more, preferably approx. 30 to 40 hours, thereby separating rabeprazole sodium salt in the crystalline hydrate form. This form can be recovered with known techniques, such as filtration or centrifugation, preferably by filtration, followed by drying under vacuum at a temperature depending on the solvent used.

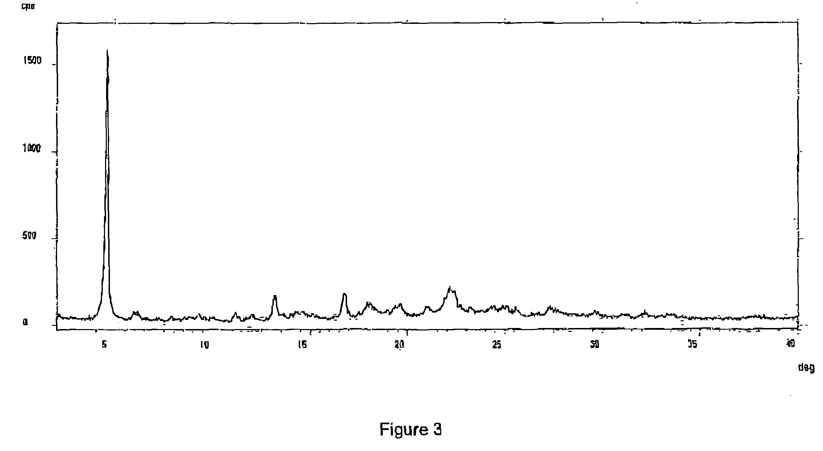

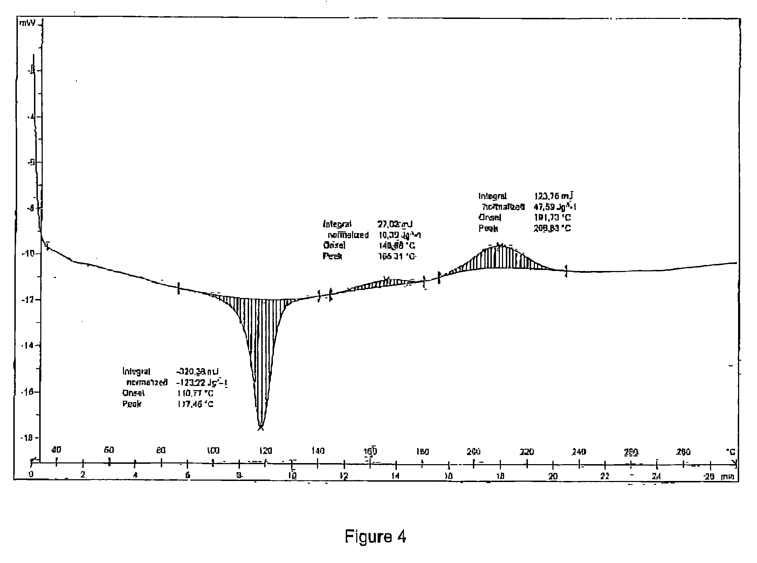

According to a second preferred aspect of the invention, an hydrate form, in the following referred to as Form β, has water content ranging between 6.0 and 7.2% in weight, preferably between approximately 6.4 and 7.0%, so that it can be defined an approximately sesquihydrate form. Said Form β, has DSC thermogram substantially as reported in Figure 4, and XRPD spectrum substantially as reported in Figure 3, wherein the more intense diffraction peaks are observed at 4.7, 9.4, 13.2, 16.8, 22.2 ± 0.2° in 2θ.

Rabeprazole sodium in the crystalline hydrate Form β, can be prepared by a process comprising:
- dissolving a rabeprazole sodium dispersion in an organic polar aprotic solvent;
- adding an alkaline water solution;
- cooling the solution at room temperature; and
- recovering the resulting solid.

The process can be carried out starting from a dispersion of rabeprazole sodium in a polar aprotic solvent. The starting crude rabeprazole sodium can be obtained as disclosed e.g. in EP 268 956. Examples and preferred exemples of aprotic polar solvents are thase mentioned above. More preferred are ethyl acetate, isopropyl acetate and butyl acetate, or mixtures thereof, in particular butyl acetate. The concentration of rabeprazole sodium in the starting solution can range approx. from 2 to 20% w/w, preferably approx. from 10 to 18% w/w. The temperature of the dispersion is then brought to a value higher than 20°C, preferably about 35-45°C, to completely dissolve rabeprazole sodium.

To the thus obtained solution an aqueous solution of an alkaline salt, preferably a sodium salt, is added. An alkaline salt can be, for example a chloride, bromide, iodide, fluoride, sulfate, hydrogensulfate, nitrate, hydroxide, carbonate, or a bicarbonate salt, preferably sodium carbonate, sodium bicarbonate or sodium hydroxide, more preferably sodium bicarbonate. The salt is dissolved in water in a concentration comprised between 2 and 20% by weight, preferably approximately between 5 and 10%.

The water solution is added in a ratio between about 5 and 15% by weight, preferably between about 7 and 13%, with respect to the rabeprazole sodium weight.

The resulting solution is cooled to room temperature and stirred till a precipitate is formed, typically from 4 to 24 hours. Rabeprazole sodium salt in the crystalline sesquihydrate form can be recovered with known techniques, such as filtration or centrifugation, preferably by filtration, followed by drying under vacuum at a temperature depending on the solvent used.

Rabeprazole sodium crystalline hydrate, in particular the Form α or Form β, analogously to commercially available rabeprazole sodium, is useful as an inhibitor of gastric secretion and can therefore be used, for example, for the treatment of peptic ulcer.

An object of the invention is also a pharmaceutical composition comprising, as the active ingredient, rabeprazole sodium salt in the crystalline hydrate form and, if desired, at least one of the known forms of rabeprazole, together with a diluent and/or carrier. Said composition preferably contains at least one of novel Form α and Form β.

Known forms of rabeprazole are for instance rabeprazole sodium salt as described in FDA NDA application No. 020973 and those ones disclosed in JP 2001-39975, WO 03/082858 A1 and US 2004/0180935.

The ratio of rabeprazole sodium salt in the crystalline hydrate form, in particular as Form α and/or Form β, to rabeprazole in one or more of the known forms will be chosen depending on their physical and biological properties and will be apparent to those skilled in the art.

The pharmaceutical compositions of the invention can be formulated in a variety of pharmaceutical forms for the administration to humans or animals, according to known techniques. Suitable formulations can be, for example, suspensions, emulsions, solutions, capsules, tablets, sugar-coated pills or other known forms. By way of example, the active ingredient unit dosage for tablets, preferably gastro-resistant, protracted-release tablets, can range from approx. 10 mg to approx. 30 mg, preferably 20 mg.

The processes reported above for the preparation of rabeprazole sodium salt in the hydrate crystalline form, particularly as Form α or Form β, allow to purify the final product from any impurities formed during the synthesis of rabeprazole, due to either parasitic reactions or degradation of the product itself.

Therefore, a further object of the present invention is a process for the purification of rabeprazole sodium salt, comprising the conversion of crude rabeprazole sodium salt, as obtainable for example according to EP 268 956, into rabeprazole sodium salt crystalline hydrate form, in particular Form α or Form β, and, if desired, its subsequent conversion into a known rabeprazole form. If desired, the starting product can also be any known rabeprazole sodium salt form.

Preferably said purification process comprises converting a rabeprazole sodium salt into rabeprazole sodium salt crystalline hydrate Form α or Form β, respectively, by a process comprising:
- dissolving a rabeprazole sodium dispersion in an organic polar aprotic solvent;
- keeping the solution at room temperature for a time equal to or higher than 24 hours; and
- recovering the resulting solid hydrate Form α; or
- dissolving a rabeprazole sodium dispersion in an organic polar aprotic solvent;
- adding an alkaline water solution;
- cooling the solution at room temperature; and
- recovering the resulting solid Formβ; and, if desired,
converting the resulting Form α or Form β into a known rabeprazole sodium salt form.

The process of the invention provides rabeprazole sodium salt, in particular as crystalline hydrate form, more precisely as Form α and Form β, in a purity of or higher than 99.9%, i.e. of suitable quality to fulfill the regulatory requirements for therapeutical products.

The following example illustrates the invention.

### Example 1. Preparation of rabeprazole Form α

2.3 g of rabeprazole sodium salt are dissolved at approx. 40°C in 30 ml of butyl acetate, The mixture is cooled to 25°C and filtered through active charcoal. The resulting clear solution is kept at 20-25°C for three days without stirring, thereby obtaining a white solid which is filtered by suction, washed with 5 ml of butyl acetate and dried under vacuum. The resulting product is a white powder that shows an XRPD spectrum substantially as reported in Figure 1 and a DSC thermogram substantially as reported in Figure 2, which show that the compound is crystalline. The water content in the compound, according to Karl Fisher, is about 2.5-2.8% in weight.

¹H NMR (300 MHz, DMSO-d6) δ (ppm): 8.26-8.24 (d, 1H); 7.48-7.44 (m, 2H); 6.92-6.87 (m, 3H); 4.70-4.66 (d, 1H); 4.45-4.41 (d, 1H); 4.09-4.05 (t, 2H); 3.48-3.44 (t, 2H); 3.23 (s, 3H); 2.14 (s, 3H); 1.99-1.93 (q, 2H).

### Example 2. Preparation of rabeprazole Form β

35 g of rabeprazole sodium salt are dissolved at approx. 40°C in 250 ml of ethyl acetate. 3 ml of an 8% aqueous solution of sodium bicarbonate are added. The mixture is cooled to 25°C and kept at 20-25°C for 4-24 hours, thereby obtaining a white solid which is filtered by suction, washed with 50 ml of ethyl acetate and dried under vacuum. The resulting product is a white powder that shows an XRPD spectrum substantially as reported in Figure 3 and a DSC thermogram substantially as reported in Figure 4, which show that the compound is crystalline. The water content in the compound, according to Karl Fisher, is about 6.4-7.0% in weight.

## Claims

1. Rabeprazole sodium, 2-[[[4-(3-methoxypropoxy)-3-methyl-2-pyridinyl]methyl]sulfinyl]-*1H*-benzilimidazole sodium salt, in the crystalline hydrate form.

2. Rabeprazole sodium Form α, according to claim 1, having a water content approx. ranging from 2.2 to 3,0% in weight.

3. Rabeprazole sodium, according to claims 1 or 2, having an XRPD spectrum wherein the more intense diffraction peaks are observed at 3.8; 5.1; 7,1; 16.9; 17.6; 18.8 and 19.9 ± 0.2° in 2θ.

4. Rabeprazole sodium Form β, according to claim 1, having a water content ranging between 6.0 and 7.2% in weight.

5. Rabeprazole sodium, according to claims 1 or 4, having an XRPD spectrum wherein the more intense diffraction peaks are observed at 4.7, 9.4, 13.2, 16.8,22.2 ± 0.2° in 2θ.

6. A pharmaceutical composition comprising, as the active ingredient, rabeprazole sodium in the crystalline hydrate form, as defined in claim 1, and, if desired, at least one of the known forms of rabeprazole, together with a diluent and/or carrier.

7. A pharmaceutical composition, according to claim 6, wherein rabeprazole sodium in the crystalline hydrate form is at least one of Form α and Form β, as defined in claims 3 and 5, respectively.

8. A process for the purification of rabeprazole sodium salt, comprising the conversion of crude rabeprazole sodium salt into rabeprazole sodium salt crystalline hydrate form, as defined in claim 1, and, if desired, its subsequent conversion into a known rabeprazole form.

9. A process, according to claim 8, comprising converting rabeprazole sodium salt into rabeprazole sodium salt crystalline hydrate Form α or Form β, as defined in claims 3 and 5, respectively, by a process comprising:
• dissolving a rabeprazole sodium dispersion in an organic polar aprotic solvent;
• keeping the solution at room temperature for a time equal to or higher than 24 hours; and
• recovering the resulting solid hydrate Form α; or
• dissolving a rabeprazole sodium dispersion in an organic polar aprotic solvent;
• adding an alkaline water solution;
• cooling the solution at room temperature; and
• recovering the resulting solid Formβ; and, if desired,
converting the resulting Form α or Form β into a known rabeprazole sodium salt form.

10. Rabeprazole sodium salt having purity of or higher than 99.9%.

11. Rabeprazole sodium salt according to claims 1, 3 or 5 having a purity of or higher than 99.9%.
